# EUROPEAN PATENT SPECIFICATION

(11) **EP 1 833 532 B1**
(45) Date of publication and mention of the grant of the patent: **23.02.2011**
(21) Application number: 05818511.7
(22) Date of filing: 22.12.2005
(51) Int. Cl.: A61M 5/19

(54) **SAFETY SYRINGE**
SICHERHEITSSPRITZE
SERINGUE DE SECURITE

(30) Priority: 24.12.2004 AU 2004907312
(43) Date of publication of application: 19.09.2007
(73) Proprietor: Collyn Patent Holdings Pty Ltd, Brighton-le-Sands, NSW 2216 (AU)
(72) Inventor: Moore, Colin Campbell Marshall, North Sydney, NSW 2060 (AU)
(74) Representative: Wakerley, Helen Rachael
(86) International application number: PCT/AU2005/001939
(87) International publication number: WO 2006/066336

(56) References cited:
- EP-A1- 1 093 826
- WO-A-96/29106
- WO-A-03/074103
- WO-A1-01/26718
- WO-A1-84/00011
- WO-A1-93/00122
- GB-A- 2 002 241
- US-A- 3 785 379
- US-A- 4 055 177
- US-A- 4 693 706
- US-A- 4 693 708
- US-A- 4 702 738
- US-A- 5 222 945
- US-A- 5 395 325
- US-A- 5 779 668
- US-A- 6 093 170
- US-A- 6 093 170
- PATENT ABSTRACTS OF JAPAN vol. 2003, no. 12 & JP 2004 097472 A (NIPRO CORP) 02 April 2004
- PATENT ABSTRACTS OF JAPAN vol. 2003, no. 12 & JP 2004 049803 A (NIPRO CORP) 19 February 2004

## Description

### FIELD OF THE INVENTION

The present invention relates to a drug delivery system and apparatus therefore. More particularly, the invention further relates to a single use syringe for delivery of a predetermined dosage of a drug and to a syringe which, in at least some embodiments, may reduce the risk of needle stick injury before and after use.

### BACKGROUND OF THE INVENTION

There is a perceived need for a syringe adapted for delivery of drugs in such a way that the syringe, when assembled, can only be used once for the delivery of a drug and cannot be refilled for subsequent uses. It is also desirable that the syringe can never be used in a reverse direction or otherwise permit reflux whereby liquids move within the syringe in a reverse direction to the delivery direction. Australian Patent No. 708445 discloses a basic syringe assembly which, in at least some embodiment, can provide this type of functionality. Specifically, this syringe assembly comprises a single sealed reservoir holding a therapeutic substance for administration to a patient and which is non-detachably connected to a needle assembly. The needle assembly incorporates a one-way valve for inhibiting return flow of the substance from the needle assembly to the reservoir during delivery of the substance. Once the syringe has been used, the one-way valve essentially prevents refilling of the reservoir.

A further matter of concern in drug delivery and, particularly, in drug delivery by way of a syringe, is that unauthorised persons may gain access to the drugs together with the syringes. It is perceived as desirable to minimise the possibility of unauthorised access to syringes and the drugs together.

A separate problem concerns the incorrect loading of dosages into syringes even by practitioners skilled in the art. Mistakes can also occur where the correct dosage is loaded into the syringe but not all of the dosage is delivered. This can happen, for example,
where over priming of the syringe occurs prior to use of the syringe resulting in an insufficient quantity of drug remaining in the syringe for actual delivery.

Moreover, a problem with at least some forms of substances intended for, for example, delivery into the human or animal body is that the substance has a limited shelf life once the substance has been prepared for use. The preparation of a substance for use can itself be a procedure requiring care and experience, especially where the procedure involves the mixing of predetermined starting substances so as to produce a final substance of predetermined characteristic such as for example having a predetermined concentration of a therapeutically effective substance. Particularly where substances are intended for injection into the human body, the volume of substance for injection and its concentration can be critical to the point where errors can lead to adverse physiological outcomes and in extreme cases, to life threatening consequences.

Yet a further problem with drug delivery syringes lies in the risk of "needle stick injury" from a syringe needle contaminated with infectious material after use. A means of lessening such risk is disclosed in United States Patent No. 4,976,702 wherein a slideable sheath is provided which may be moved from a retracted position substantially coextensive with the barrel of the syringe to an extended position so as to cover the needle. However, this arrangement relies on retention of the sheath in either of its positions on the barrel, and on the engagement of internal protuberances within the sheath with annular grooves around the barrel. Only an axially directed force needs be applied to the sheath to move it between extended and retracted positions. Hence, this arrangement does not effectively lock the sheath in the extended position and cannot prevent the needle from contacting and puncturing the skin if the sheath is accidentally pushed against it with a similar force to that required to operate the sheath.

UK Patent document GB 2002241 discloses a syringe having a fluid medicament and a diluent sealed in two separate telescoping syringe barrels, each of which feature a pierceable seal. A double-pointed cannular 29 is positioned between the two pierceable seals 20 and 32 by means of a telescoping guide arrangement. Inward movement of the plunger rod 51 initially causes a guide block 41 to be displaced due to frictional engagement between the guide block 41 and a tubular guide means 36. This initial retention causes point 38 of the cannular 29 to pierce seal 20. Once guide 30 abuts stop portion 66 of bushing 26, the frictional engagement between the guide block 41 and the tubular guide means 36 is overcome and the relative movement of the cannular 29 ceases. This subsequent sliding between the guide block 41 and the tubular guide means 36 allows the point 37 of the cannular 29 to pierce seal 32 upon continued inward movement of the plunger rod 51. Once the double-pointed cannular 29 has pierced both of the seals 20 and 32 the fluid medicament and the diluent intermix. Further movement of the plunger expels the mixed medicament from the syringe.

### SUMMARY OF THE INVENTION

In one aspect of the present invention there is provided a multi-compartment syringe adapted for storage and subsequent ejection of a mixed substance from an exit portion thereof, the multi-compartment syringe comprising:
a first compartment adapted for sealed storage of a first substance;
a second compartment adapted for sealed storage of a second substance;
communication means having a sealed state and a communicating substance state, the communication means comprising a piercing element arranged for piercing a slidable sealing element;
communication actuation means adapted to move the communication means from the sealed state to the communicating state;
first urging means by which the second substance in at least the second compartment, is urged from the second compartment into the first compartment so as to mix with the first substance thereby to form the mixed substance; and
second urging means by which the mixed substance in the first compartment is urged through the exit portion, characterised in that the piercing element has a single tip arranged for piercing a single slidable sealing element and also being
characterised in that the communication means further includes a breakable seal for sealing substance flow through the piercing means, whereby, when in the communicating substance state, the slidable seal is pierced by the piercing element and the breakable seal is broken so as to permit the second compartment to be placed in substance communication with the first compartment.

Typically, the first and second urging means will comprise a single plunger aligned with a longitudinal axis of the syringe, the plunger in a first range of movement acting as the first urging means, and the plunger in a second range of movement acting as the second urging means.

In another aspect of the present invention there is provided a method for operating a multi-compartment syringe, comprising:
providing a first cylindrical storage means and a second cylindrical storage means, the second storage means being received by the first storage means and having a single tip piercing element at one end region thereof and a slidable sealing element at an opposite second end region thereof, and a breakable seal sealing the piercing element from an internal storage volume of the second storage means;
driving the second storage means axially such that the single tip piercing element pierces a single sealing element of the first cylindrical storage means;
driving the sealing element of the second cylindrical storage means axially to breach the breakable seal of the second storage means thereby placing the first and second storage means in fluid communication for discharge of contents of the second storage means into the first storage means; and
discharging the contents of the second storage means into the first storage means to provide a mixed substance.

Preferred aspects of the invention are set out in the accompanying claims.

Typically, the piercing element of a syringe or syringe assembly embodied by the invention will comprise a hollow needle.

Typically, in multi-compartment syringes embodied by the invention, the contents of the first storage means will be different to the contents of the second storage means. Most preferably, the contents of the first storage means will comprise a powder and the contents of the second storage means will comprise a liquid for forming a solution with the powder. By providing the substances in the first and second storage means separately from one another until use, the shelf life of one or both of the substances may be increased compared to the resulting mixture of the substances when combined together.

A syringe or syringe assembly embodied by the invention will generally comprise a single use syringe adapted for a single delivery of the mixed substance. The mixed substance may comprise one or more therapeutic agents. The agent(s) may, for instance, be selected from the group consisting of drugs, vitamins, nutrients, other substance for providing a therapeutic outcome, and mixtures of the foregoing.

In a related embodiment there is provided a needle assembly suitable for use with a syringe embodied by the present invention.

In further related embodiment there is provided a one-way valve system for use with a syringe embodied by the invention.

In another related embodiment there is provided an adaptor sleeve for connecting the needle assembly of a syringe embodied by the present invention to a cylindrical storage means as described above.

In yet another related embodiment there is provided a single use syringe, the syringe comprising self-contained storage means adapted to sealingly retain a predetermined dosage of a therapeutic substance, the storage means being adapted to engage an actuator at a first end thereof and delivery means at an opposite end region thereof.

In a still further related embodiment there is provided a system for the safe delivery of a drug or drugs from storage means adapted for independent storage from delivery means used for the delivery of the drug or drugs. Preferably, the delivery means is provided separately to the user.

Most preferably, each component part of the syringe including the cylindrical storage means is unusable for drug administration in its own right unless and until assembled together to form a syringe of the present invention by a conscious act.

Any discussion of documents, acts, materials, devices, articles or the like which has been included in this specification is solely for the purpose of providing a context for the present invention. It is not to be taken as an admission that any or all of these matters form part of the prior art base or were common general knowledge in the field relevant to the present invention as it existed in Australia or elsewhere before the priority date of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The features and advantages of the present invention will become further apparent from the following detailed description of preferred embodiments together with the accompanying drawings.

### BRIEF DESCRIPTION OF THE ACCOMPANYING DRAWINGS

**Figure 1** is an exploded side exploded schematic view of a single use syringe assembly;
**Figures 2A-2I** illustrate steps for the assembly and use of the syringe assembly of Fig. 1 for drug delivery;
**Figures 3A-3L** illustrate further steps in the assembly and use of the syringe assembly of Fig. 1;
**Figure 4** is a block diagram of a drug delivery system;
**Figure 5** is a schematic view of a kit for a syringe suitable for use in the drug delivery system illustrated by Fig. 4;
**Figure 6** shows side sectional views of a multi-compartment syringe embodied by the invention at four separate stages of use;
**Figure 7** shows front views of a safety sheath of a syringe assembly in extended and retracted positions when fitted to a syringe according to Fig. 1 to Fig. 3L;
**Figure 8** is a partial side sectional view of the syringe of Fig. 7 showing a safety sheath locking arrangement with the sheath in an extended position;
**Figure 9** is a perspective view of the syringe and safety sheath of Fig. 7 and Fig. 8;
**Figure 10** shows front views of a further embodiment of the safety sheath of Figs. 7 to 9.

### DETAILED DESCRIPTION OF PREFERRED EMBODIMENTS OF THE INVENTION

In the description that follows, the term "liquid" includes any fluid substances stored in or ultimately delivered by the syringe assembly. It is to be understood that the term encompasses, for example, single fluids, emulsions, suspensions, an admixture of a fluid and a solid suspension, an admixture of a plurality of liquids, and combinations of the foregoing.

### First Embodiment

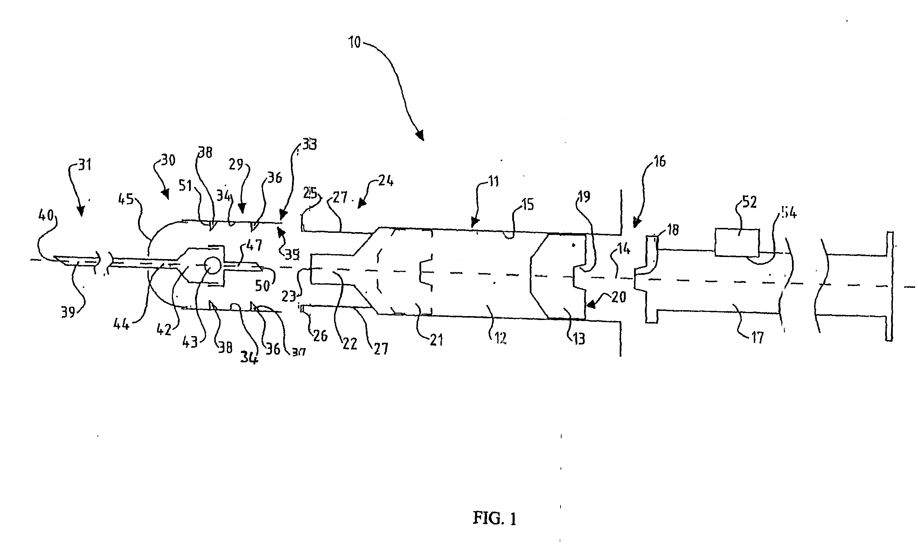

Fig. 1 is a schematic of a single use syringe assembly 10. In this instance the syringe assembly 10 includes a single self-contained, cylindrical storage means in the form of a barrel-shaped container 11 having a storage volume 12 defined therein. The precise volume of the storage volume 12 is determined by a seal 13 sealingly and slidably moveable along longitudinal axis 14 of container 11 and, which at all times during movement, retains sealing contact with inner wall 15 of container 11. First end 16 of container 11 is adapted to slidingly receive therein an elongate plunger 17. The plunger 17 is adapted to be aligned along its longitudinal axis with axis 14 of container 11 and to be advanced into first end 16 of container 11 such that seal connector 18 at a leading edge thereof, seats releasably within plunger connector seat 19 located in rear surface 20 of moveable seal 13. The plunger connector seat 19 is in the form of a cavity adapted to releasably receive seal connector 18 in the form of a boss or dowel pin. The seating of the non-retaining seal connector 18 within the plunger connector seat 19 is such that the plunger 17 can be withdrawn from its engagement with the moveable seal 13 simply by reversing the movement of plunger 17 so that it moves out of container 11 and away from first end 16 along longitudinal axis 14 and leaving the moveable seal 13 retained within container 11 at the position it had assumed when the direction of movement of plunger 17 was reversed.

The plunger 17 is of sufficient length that it may be utilised to urge moveable seal 13 along longitudinal axis 14 all the way to an end position 21 within container 11 which, in so doing, forces the contents in storage volume 12 in the direction of container outlet 22. The container 11 includes a frangible seal in the form of a membrane 23 which, for transport of container 11, seals container outlet 22 thereby to prevent egress, during transport, of liquid within storage volume 12.

Second end 24 (the delivery end) of container 11 also incorporates delivery means engagement means in the form of first engagement tyne 25 and second engagement tyne 26, both mounted on substantially cylindrical tyne support unit 27 which itself extends from second end 24 of container 11.

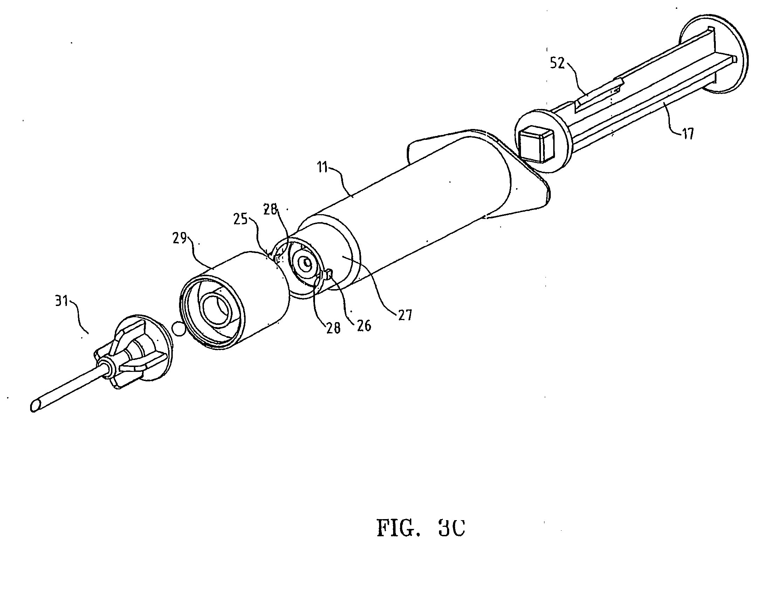

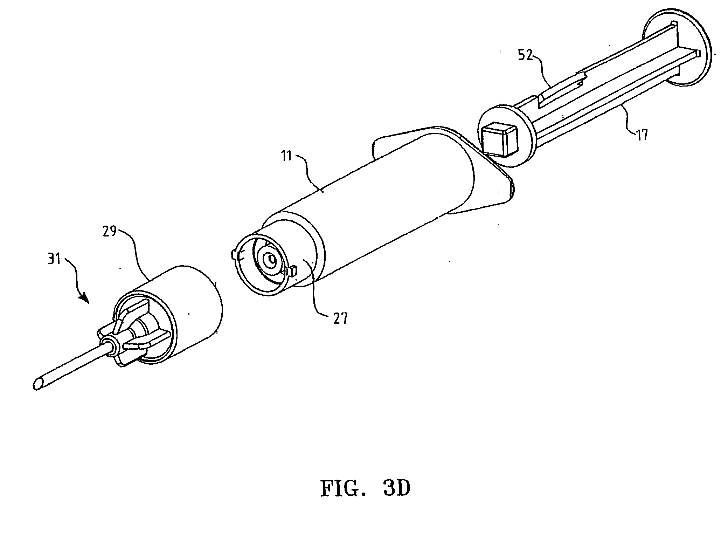

With reference to Figs. 3C and 3D, the tyne support unit 27 includes opposed slots 28 therein which are of sufficient width to allow at least limited flexing inwardly of tynes 25, 26 for the purpose of assisting the engagement of the tynes 25, 26 with the delivery means as described further below. The delivery means comprises an adapter sleeve 29 that receives at a first or delivery end 30 thereof a delivery device such as a needle assembly 31 or an infusion connector 32 for connection to another delivery system as can be performed for example by a Luer-type fitting. The adapter sleeve 29 at a second end or receiving end 33 thereof is adapted to non-releasably engage with the delivery means engagement means of the container 11 which, in this instance, comprises the engagement tynes 25, 26 and the container outlet 22.

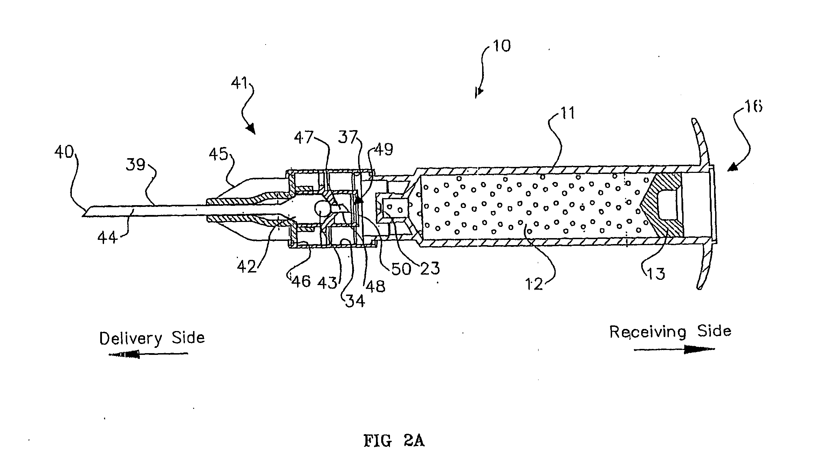

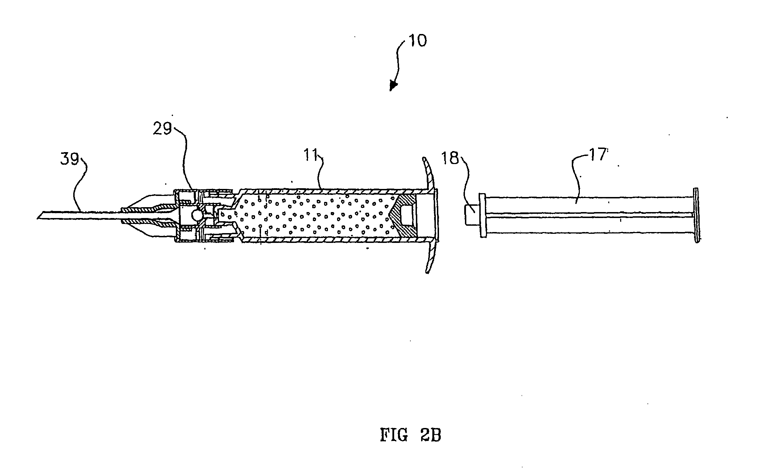

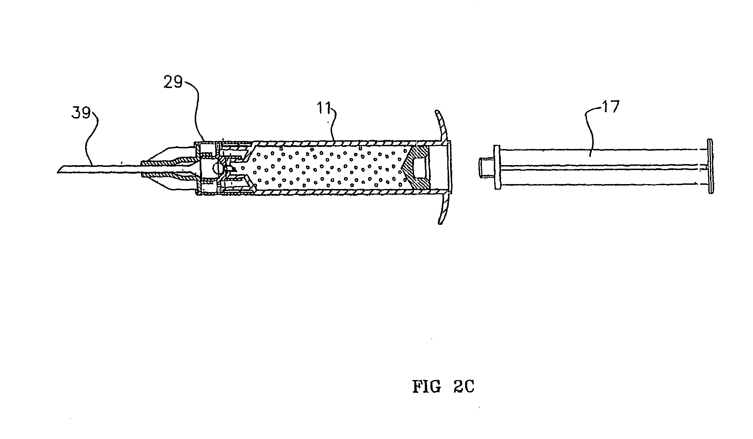

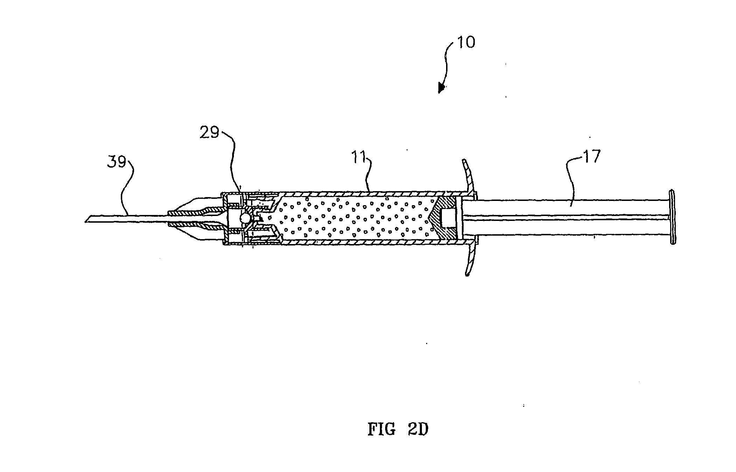

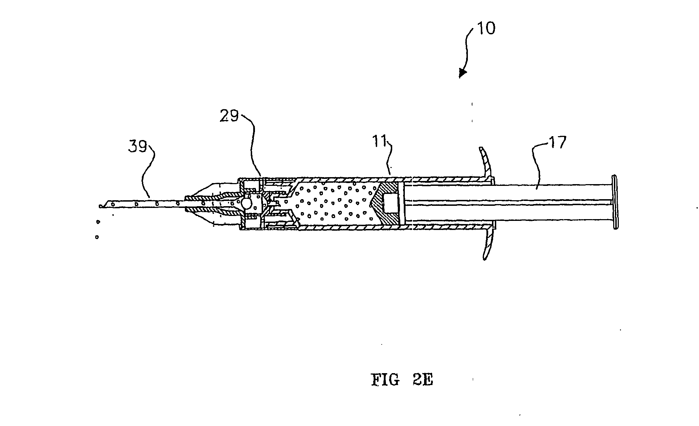

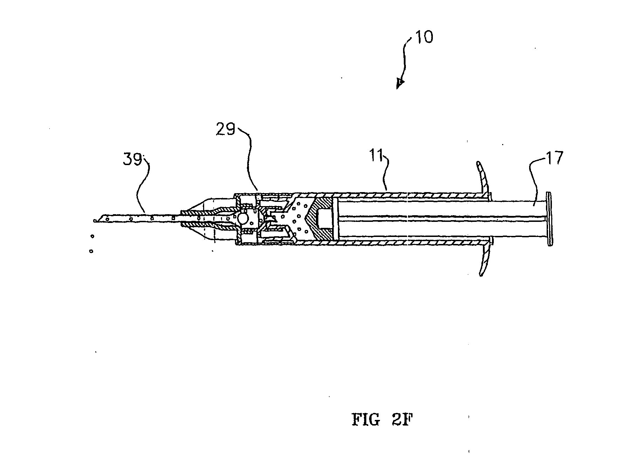

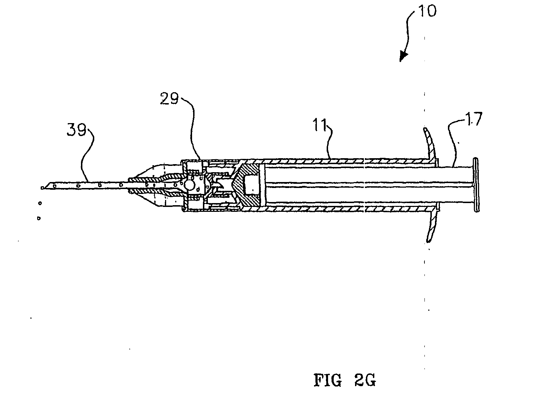

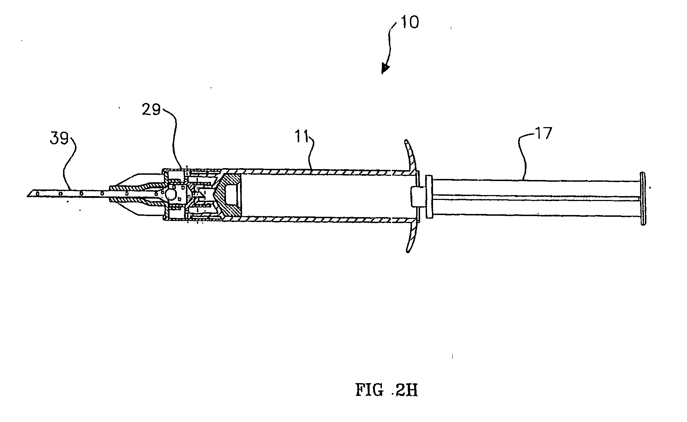

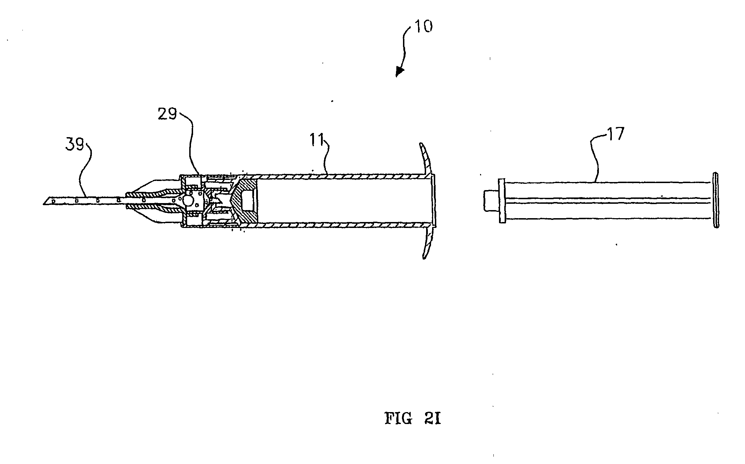

The tynes 25, 26 engage in a spiral shaped channel 34 located on or in the inside wall 35 of adapter sleeve 29 and progress along the channel as and when the container 11 is twisted relative to the sleeve 29 until they encounter first tyne barriers 36. At this point, further engagement of the container 11 with the adapter sleeve 29 is achieved by a forward urging of container 11 along longitudinal axis 14 in the direction of sleeve 29 whereby the tynes 25, 26 ride up and over inclined surfaces 37 (see Fig. 1 and Fig. 2A) of barriers 36 to permit continued travel of the tynes 25, 26 in channel 34 upon continued twisting movement of container 11 relative to sleeve 29, the spiral channel 34 and the tyne barriers 36, comprising spiral-action locking means. In this embodiment, second tyne barriers 38 forming a second stage of the spiral-action locking means are disposed further along channel 34 from the first tyne barriers 36, which barriers must also be surmounted by tynes 25, 26 in the manner described above in relation to the first tyne barriers 36 before final and complete, sealing and non-releasable engagement of container 11 is achieved with adapter sleeve 29.

The delivery means includes, in addition to the sleeve 29, the needle assembly 31 engaged within the first end or delivery end 30 of the adapter sleeve 29. Specifically, the needle assembly 31 (see Fig. 1 and Fig. 2A) comprises elongate needle 39 having a contoured needle tip 40 at a delivery end thereof adapted for facilitating insertion of the needle into the tissue of a patient.

At a receiving end 41 of the needle 39 is a bulbous chamber 42 receiving valve ball 43 therewithin. The bulbous chamber 42 has sufficient volume compared with the dimensions of the valve ball 43 and is configured such that the ball valve 43 cannot impede forward progress of a liquid through chamber 42 in the direction of elongate delivery channel 44 within needle 39.

Needle housing 45 grips receiving end 41 of needle 39 and includes adapter sleeve engagement tynes 46 which, upon insertion of the receiving end 41 of needle assembly 31 into the delivery end of adapter sleeve 29, mechanically engages with the adapter sleeve 29 so that the receiving end 41 of the needle assembly 31 is retained within adapter sleeve 29 in a manner whereby delivery channel 44 including bulbous chamber 42 are placed in liquid communication with the delivery end of adapter delivery tube 47 which is housed within and forms part of adapter sleeve 29. In addition, the engagement thus described causes the bulbous chamber 42 in conjunction with valve ball 43 to act as a one way valve in that valve ball 43 is sized so as to substantially impede if not entirely exclude reflux of liquid within chamber 42 into adapter delivery tube 47. This action is achieved by mechanical sealing of the surface of valve ball 43 against delivery end 48 of adapter delivery tube 47 as ball 43 is urged by the reverse flow of liquid against delivery end 48. This one-way or anti-reflux function can be assisted by biasing the ball in the direction of delivery end 48, for example by use of biasing means such as a spring.

Receiving end 49 of adapter delivery tube 47 is profiled so as to include a sharp apex 50 adapted to pierce membrane 23 during engagement of adapter sleeve 29 with container 11 to provide a channel of liquid communication from storage volume 12 of container 11 through adapter delivery tube 47, through bulbous chamber 42 and through needle delivery channel 44 in the needle tip 40.

It will be appreciated by those skilled in the art that at least adapter sleeve 29 is rendered difficult to disengage from container 11 by ledges 51 on the delivery side of barriers 36, 38 which are in planar engagement with corresponding ledges 52 on the receiving side of engagement tynes 25, 26.

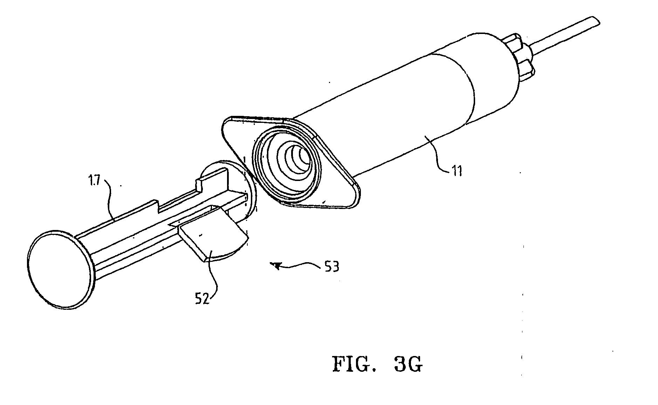

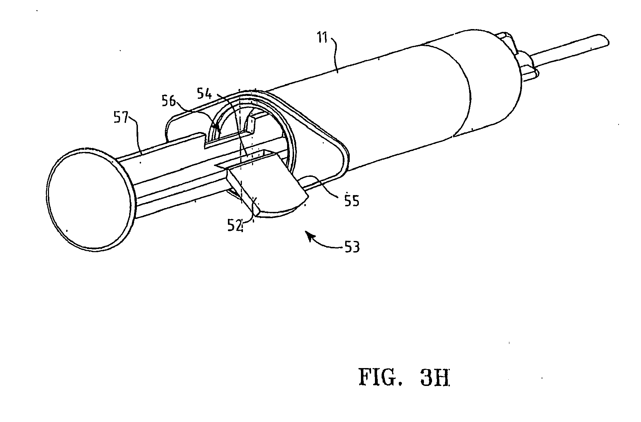

In a particularly preferred form, plunger 17 includes moveable barrier 52 which is pivotally connected to plunger 17 so as to perform the function, in an arresting position, of a pre-load arrestor that prevents sliding movement of plunger 17 into container 11 beyond a predetermined insertion limit. The moveable barrier 52 is shown in its arresting position 53 in Figs. 3G and 3H.

The moveable barrier 52 can be rotated about hinge line 54 so that its leading (arresting) edge 55 lies within slot 56 of plunger body 57 in a non-arresting position where it no longer engages with first end 16 of container 11, permitting plunger body 57 to progress into container 11 so as to cause moveable seal 13 to shrink storage volume 12 and urge liquid contained therein through container outlet 22, adapter delivery tube 47, bulbous chamber 42, delivery channel 44 and out of tip 40 of needle 39.

### Second Embodiment

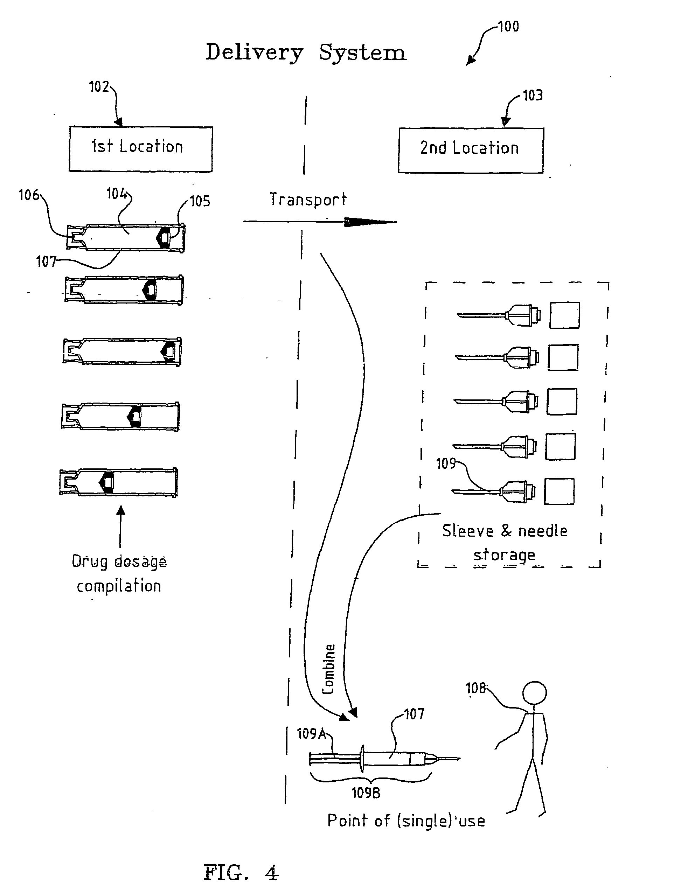

With reference to Fig. 4 there is illustrated a drug delivery system 100 based on the first embodiment of the syringe described above. The system 100 is adapted for transmission of a predetermined quantity of a drug from a first location 102 to a second location 103.

At the first location 102 the drug dosage is compiled into a discrete quantity suitable for delivery as a single dosage, for example by way of injection into a mammalian body. This predetermined quantity is then loaded into a self contained storage means in the form of a barrel shaped container 104. The barrel shaped container has actuator engagement means in the form of a moveable seal 105 at a first end thereof and, further, delivery means engagement means in the form of sealed, frangible outlet 106 at a second end thereof.

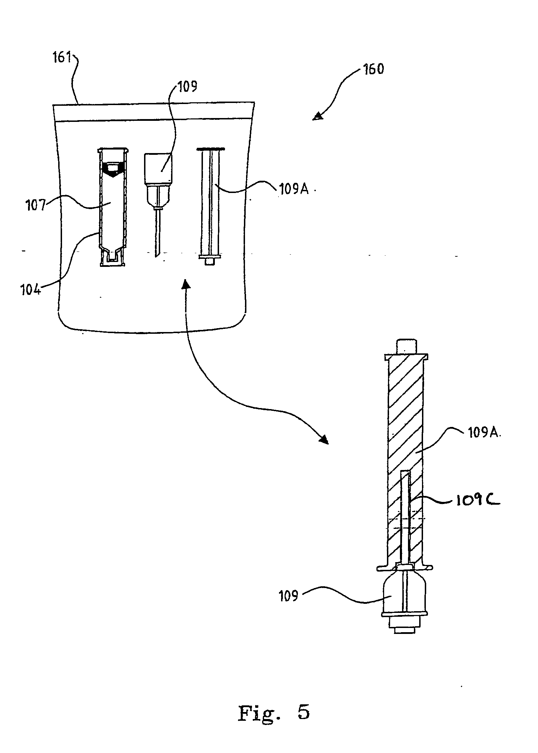

When it is required to deliver the predetermined quantity 107 of drug or other substance for example to a patient 108 by way of injection at second location 103, the container 104 is transported to the second location where it is attached to it an appropriate actuator means in the form of needle assembly 109 and an appropriate actuation means in the form of plunger 109A. After priming of the completed syringe assembly 109B injection of the predetermined quantity 107 of drug into patient 108 takes place. After use the syringe assembly 109B can be deactivated and made safe by, for example, inserting at least the needle portion of needle assembly 109 into a receiving aperture 109C in plunger 109A as illustrated in Fig. 5.

Again with reference to Fig. 5, the delivery system thus described can be provided in one form as a kit 160, in this instance a sealable plastic bag 161 into which container 104, plunger 109A and needle assembly 109 are inserted. For safety, needle assembly 109 could be stored in aperture 109C of plunger 109A as shown in the insert of Fig. 5.

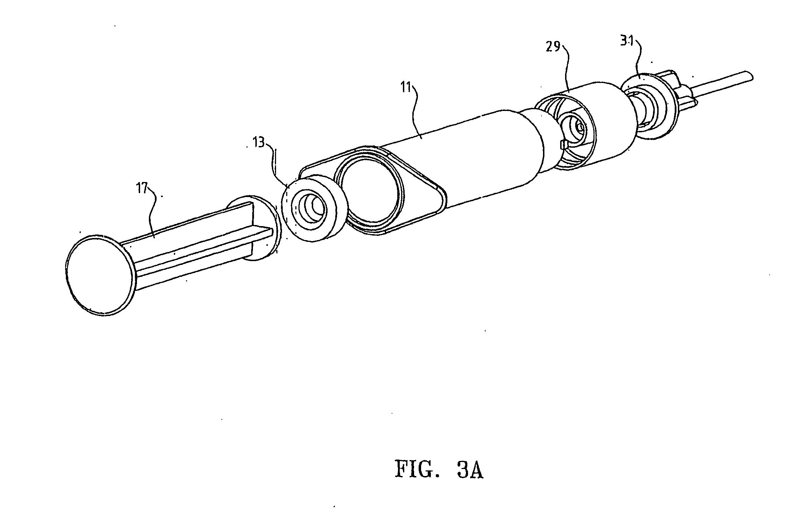

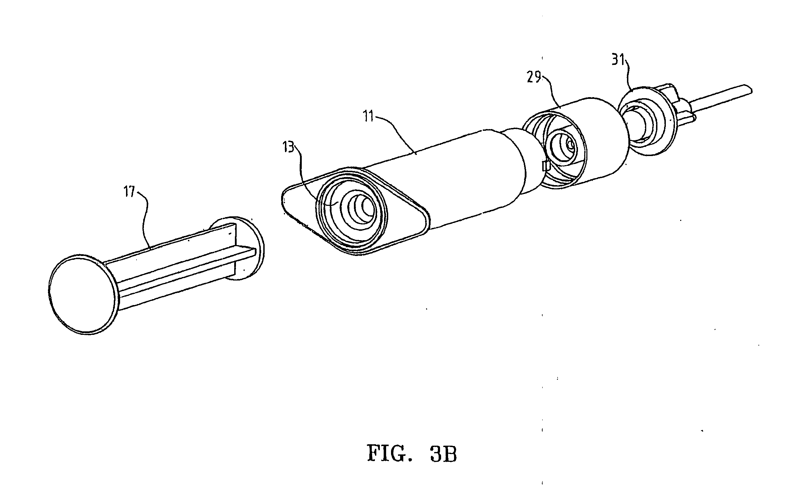

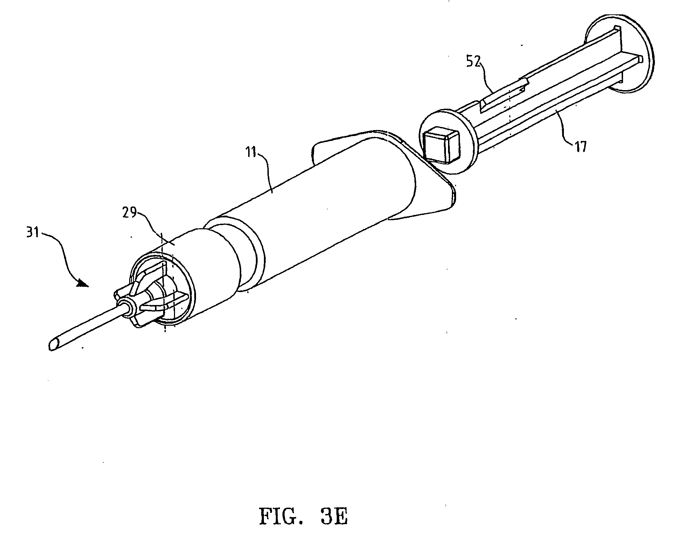

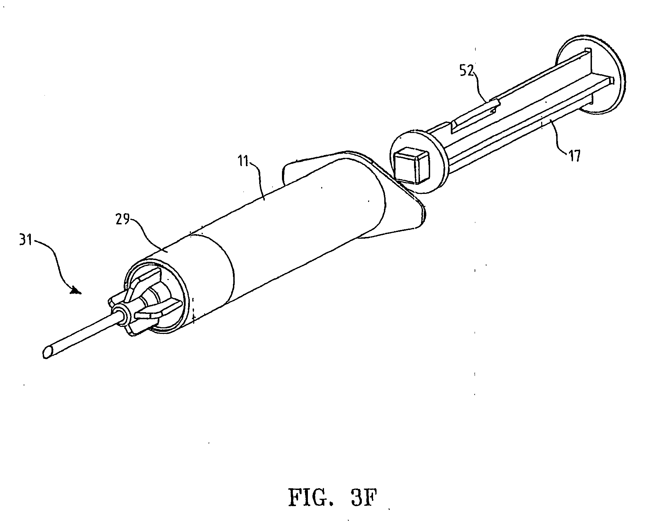

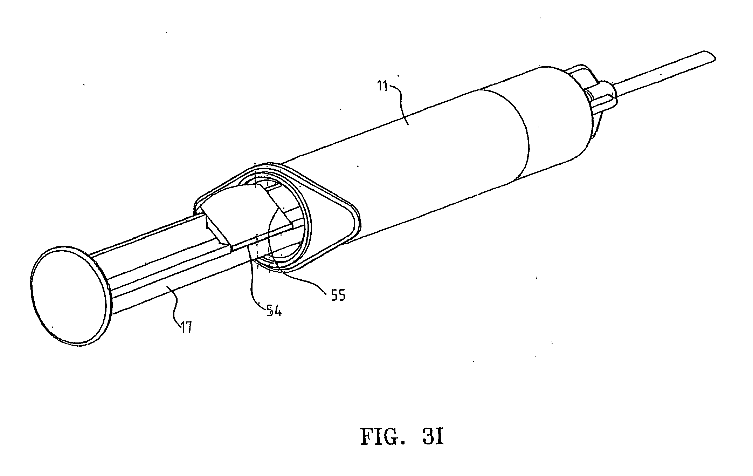

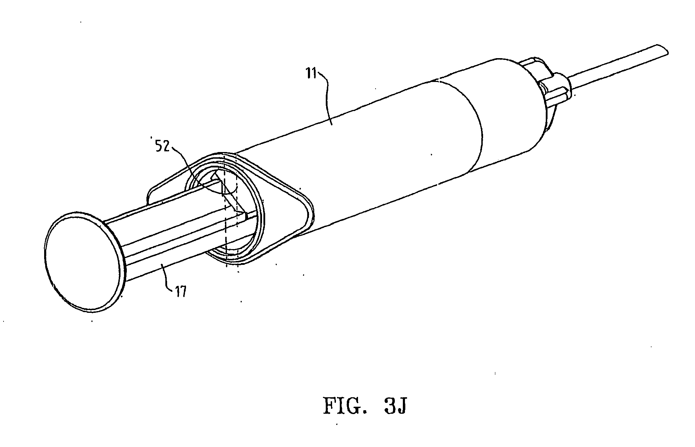

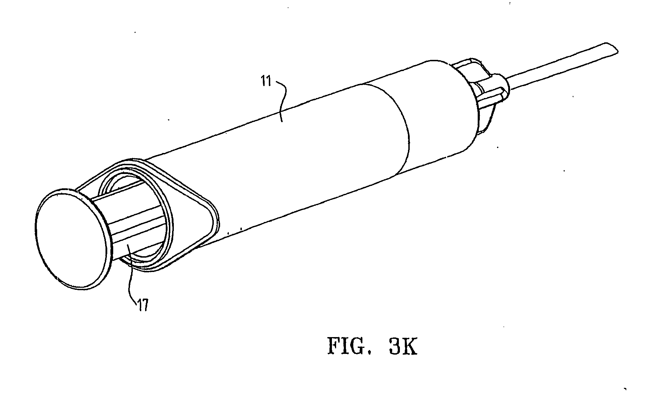

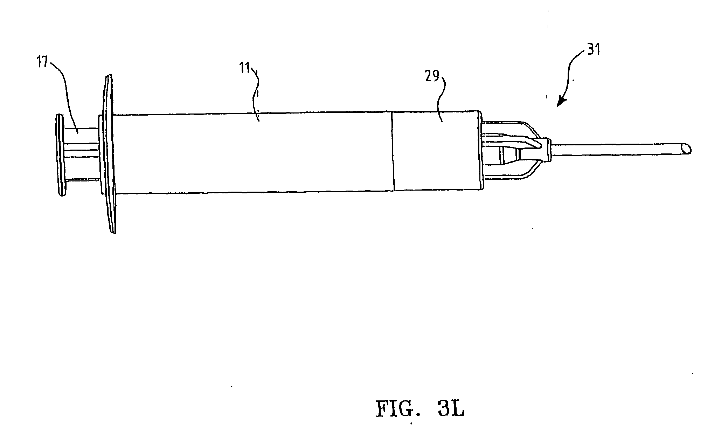

Upon transport to a location where it is desired to inject the predetermined quantity 107 of drug from the container 104, a user first removes the needle assembly 109 from within aperture 109C of plunger 109A, then assembles the syringe assembly in the manner previously described and injects the contents of container 104 in the manner previously described, for example in detail with reference to Figs. 3 and 4. The user then disassembles the syringe assembly and can render safe the sharps portion of the needle assembly 109 by insertion of the needle portion of the needle assembly 109 into aperture 109C of plunger 109A.

Where the single use syringe assembly of 109B as described above is utilised, it will be appreciated that an increased level of safety is achieved by virtue of the features of the syringe assembly, either alone or in combination, which render the container 111 that embodiment difficult if not impossible to re-use.

### Third Embodiment

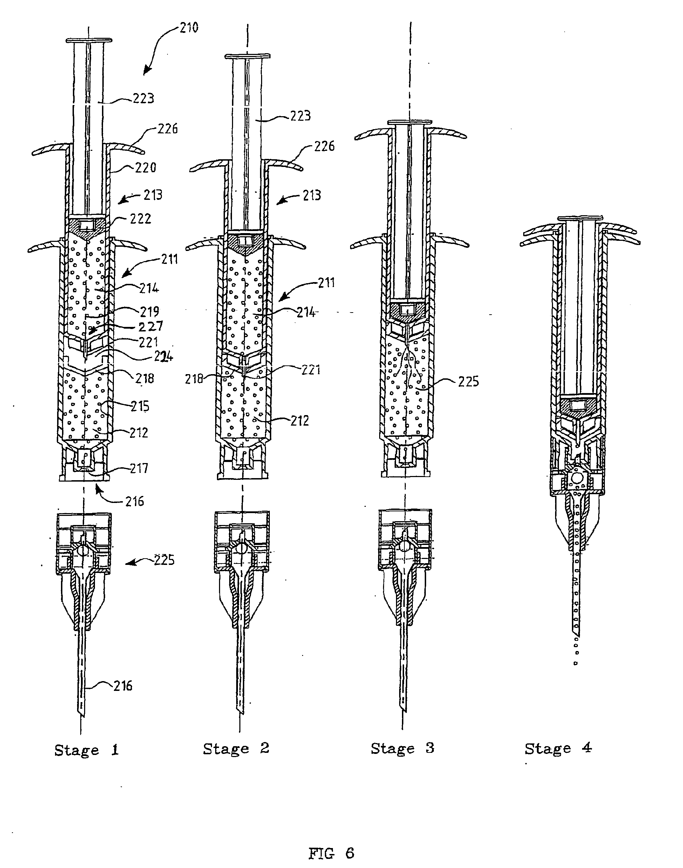

With reference to Fig. 6, there is illustrated a multi-compartment syringe 210 which, with particular reference to Stage 1 as illustrated at the left of the diagram, is shown in a pre-use or storage position and comprises at least a first self contained, cylindrical priming storage means comprising a first compartment 211 having a first substance 212 sealingly stored therein and a second self contained, cylindrical storage means comprising compartment 213 having a second substance 214 sealingly stored therein. First compartment 211 is substantially formed by a barrel portion of the syringe housing as described above, and is sealed at exit portion 216 by frangible barrier 217 and further defined and sealed at a second end by sliding seal 218 so as to provide between them a predefined volume containing a predetermined amount of a first substance 212. Also as described above, sliding seal 218 is slidingly moveable along the longitudinal axis 219 of the first compartment 211 whilst maintaining a sealing relationship between it and the interior walls of the compartment 211.

In this instance, second compartment 213, in effect replaces the plunger of the above described embodiments, and is substantially defined by an inner barrel 220 which is slidable within the barrel of first compartment 211 along longitudinal axis 219 thereof. Inner barrel 220 fits snuggly but slidingly within the syringe housing so as to be supported by and guided by the walls 215 of the syringe housing.

In the pre-use or storage position illustrated in Stage 1 of Fig. 6, second substance 214 is sealed within second compartment 213 at a first end by communication means which, in this instance, comprises a piercing needle 221 housed within first compartment 211 and at a second end by a plunger seal 222 mounted for sliding, sealing movement along longitudinal axis 219 within inner barrel 220. A breakable seal in the form of friable membrane 227 seals the inner end of hollow needle 221.

Plunger seal 222 may be attached to a working end of actuator means in the form of plunger 223, but may in at least one preferred form of the invention releasably engage with plunger seal 222 as described in the first and second embodiments above rendering the syringe single use only. Plunger 223 is adapted for sliding movement along longitudinal axis 219 within inner barrel 220. In turn, inner barrel 220 is adapted for sliding movement along longitudinal axis 219 within first compartment 211 as above described.

In this instance, the communication means of inner barrel 220 includes hollow needle 221 having a sealed state by means of a breakable seal in the form of friable membrane 227 as illustrated in Stage 1 of the pre-use or storage position of the multi-compartment syringe 210. The sealed state is assisted by the tip of the projecting piercing needle 221 being disposed in an intermediate volume 224 defined between sliding seal 218 and the first or leading end of the second compartment 213.

The hollow needle 221 is adapted to be moved along longitudinal axis 219 to a communicating substance state wherein it pierces sliding seal 218 entirely therethrough so as to form a conduit from second compartment 213 through to first compartment 211.

In use of the multi-compartment syringe 210, the syringe is initially pre-filled at the time of assembly into the pre-use or storage position illustrated in Stage 1 of Fig. 6, with a first substance 212 in first compartment 211 and a second substance 214 in second compartment 213, and with the first and second compartments in the relative positions as shown in Stage 1. Delivery means 225 for piercing the frangible seal 217 and delivery of the contents of the syringe to a patient is otherwise assembled and supplied as a separate item. The first substance 212 and second substance 214 are substances selected so that, when mixed, they will form a mixed substance 225 adapted ultimately for delivery from exit portion 216 of syringe 210.

Typically, although not exclusively, first substance 212 may be stored in a powder state or granular state within first compartment 211 whilst second compartment 213 will contain second substance 214 in the form of a solvent and distribution liquid which, when mixed with the first substance 212 will cause first substance 212 to dissolve within, or mix with, the second substance 214 so as to form a mixed substance. Of course, either one or both of the first and second compartments may contain one or more active agents (eg drugs), respectively. It will be understood that, in this context, the resulting "mixed substance" may include a suspension of one substance within another, a simple mixture of two substances and, in other instances, a homogenous liquid where one substance dissolves entirely within the other and/or a chemical reaction takes place to form an entirely new substance derived from but having a different chemical composition to that of either of the substances from which it is formed on mixture. In a particularly advantageous although by no means limiting form, the first and second substances in their separated, sealed states will have a longer shelf life than that of the substances once mixed so as to form the mixed substance. The method of use of the multi-compartment syringe will now be described.

### Stage 1

When, for example, a medical practitioner wishes to inject the mixed substance into a patient, the practitioner (not shown) takes the multi-compartment syringe 210 whilst still in its pre-use or storage position of Stage 1 and performs the following actions:

### Stage 2

The practitioner urges inner barrel 220 along longitudinal axis 219 in the direction of first compartment 211 by means of finger grips 226, sufficient to cause the communication means to change state from a sealed state to a communicating substance state by virtue of hollow needle 221 fully piercing sliding seal 218 and, in this instance, reducing the volume of intermediate volume 224 substantially to zero.

### Stage 3

Plunger 223 is now driven into second compartment 213, the pressure thus created within the contents of the compartment rupturing the friable membrane 217. Having opened a communication channel between second compartment 213 and first compartment 211, the practitioner can now urge the second substance 214 from second compartment 213 and into the first compartment 211 through hollow needle 221 by urging plunger 223 along longitudinal axis 219 in the direction of first compartment 211 to reduce the volume of second compartment 213 substantially to zero and, in so doing, cause the expulsion of the second substance 214 substantially entirely into first compartment 211. The first substance 212 and second substance 214 thereby intermingle so as to produce the mixed substance.

### Stage 4

Finally, and preparatory to expulsion of mixed substance through exit portion 216 the practitioner, if it has not already been done, will now attach delivery means comprising a suitable delivery module 225 to exit portion 216. In this example, the delivery module 225 may be a needle assembly as described in the first preferred embodiment above to pierce the frangible seal 217 so as to form a communicating path from first compartment 211 to the patient. However, as will be understood, any other form of delivery module may also be utilised. For instance, a delivery module may not be provided with a ball valve 43. Moreover, the delivery module may simply be detachably received by the syringe housing in a sliding fit such that the needle of the delivery module pierces the frangible membrane 217. Once the needle portion is inserted into the patient, the practitioner urges plunger 223 further along longitudinal axis 219 in the direction of exit portion 216 whereby the volume of first compartment 211 is reduced substantially to zero resulting in the expulsion of mixed substance 225 through exit portion 216.

### Fourth Embodiment

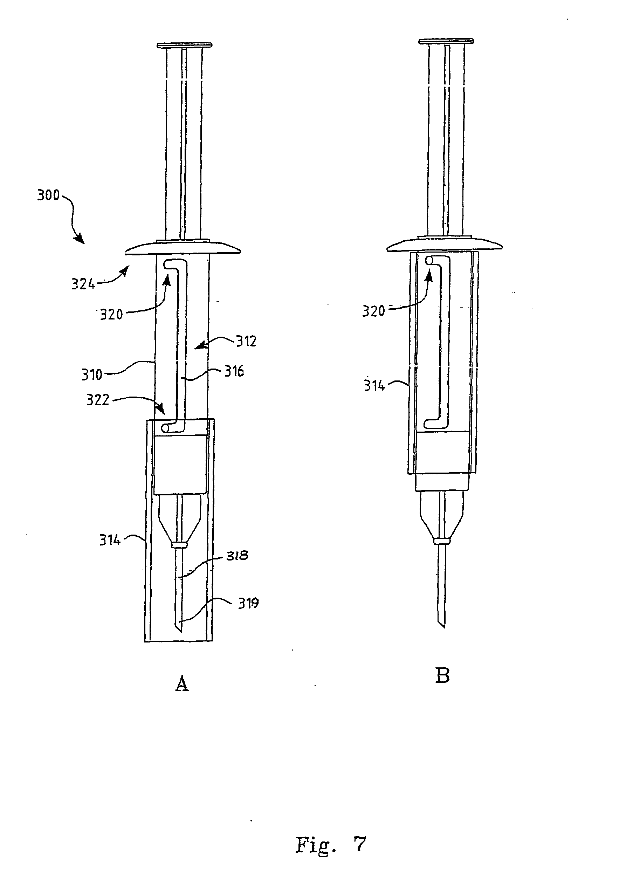

Any of the above described embodiments may be provided with a safety sheath arranged so as to prevent inadvertent needle-stick injuries. Thus for example with reference to Fig. 7, the barrel portion 310 of a syringe 300 according to the first preferred embodiment described above, is provided with a guide channel 312 for a retractable safety sheath 314. The guide channel 312 comprises a guide section 316 extending at least sufficiently along the length of the syringe barrel portion 310 for the safety sheath 314 to move between a fully retracted position when the syringe is in use, as may be seen in Fig. 7(A), and an extended position at which the safety sheath 314 extends beyond the point 319 of the needle 318 so as to render the point 319 incapable of contact with the skin of a patient or user, as shown in Fig. 7(B).

The guide channel 312 is further provided with a retracted locking segment 320 and an extended locking segment 322. Both segments are a continuation of the guide section 316 but are disposed transverse to it so as to extend a short distance around the circumference of the barrel portion 310, with the retracted locking segment 320 close to the first end 324 of barrel portion 310.

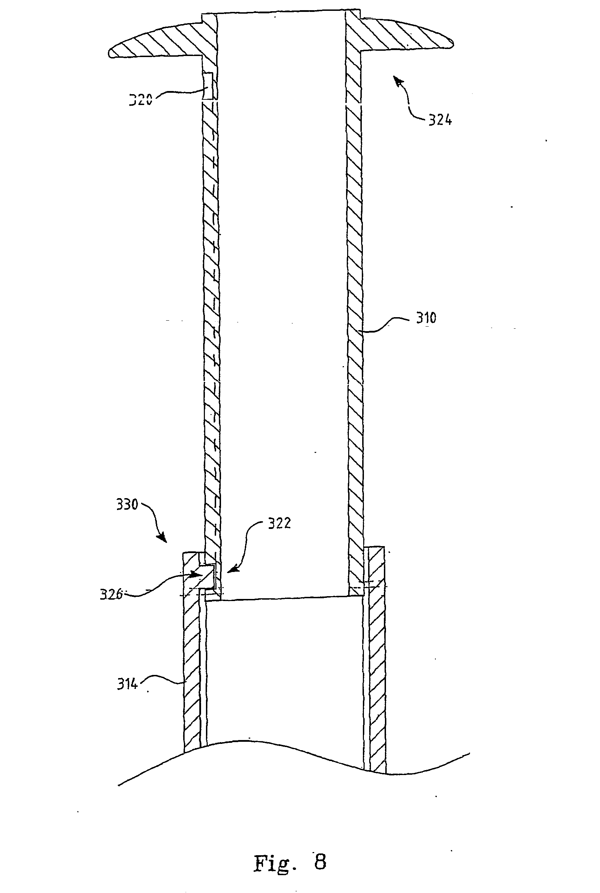

As may best be seen in the enlarged part sectioned view of the barrel portion 310 in Fig. 8, sheath 314 is provided with a generally cylindrical follower 326. The cylindrical follower protrudes radially from the inside surface 328 of the sheath 314 and is dimensioned to slide in the guide channel 316 and locking segments 320 and 322 of the syringe barrel portion 310. Further, the follower 326 is disposed near the proximal end 330 of the sheath and is arranged so that when the sheath is fully retracted, it may be rotated relative to the barrel so that the follower enters locking segments 320.

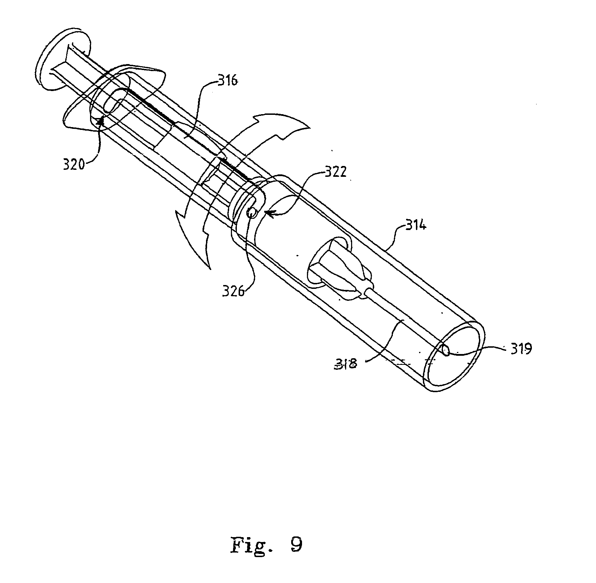

As best seen in Fig. 9, when fully expended, the sheath 314 may be rotated to enter the follower 326 into the extended locking segment 322, thereby locking the sheath in the extended position. When the follower 326 is so engaged in the locking segment 322, the sheath 314 is prevented from axial movement relative to the barrel portion 310. The protruding follower 326 and guide and locking segments 320 and 322 of the channel 312 are so sized that, particularly when in the extended position, the sheath cannot be accidentally dislodged by the sort of accidental end impacts encountered when manipulating a syringe in use.

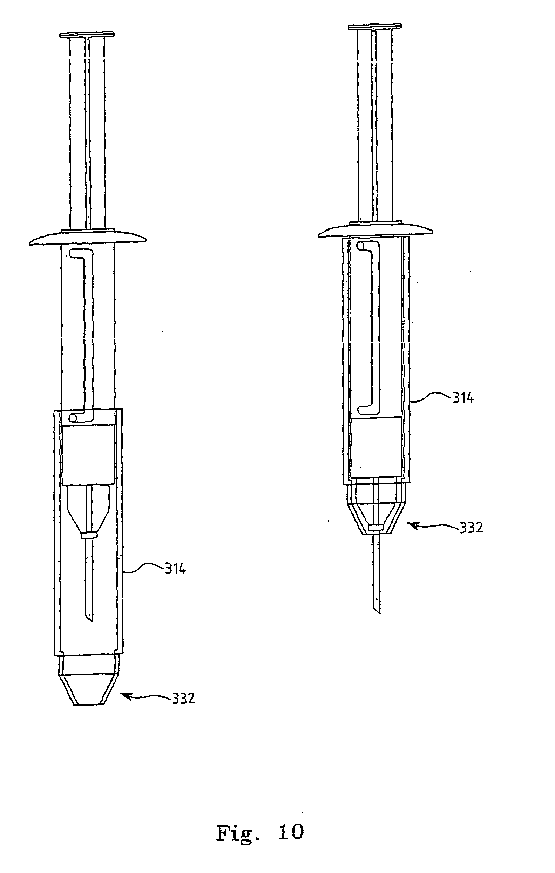

In at least one preferred embodiment of the sheath as shown in Fig. 10, the outer end 332 of the sheath 314 is extended and tapered, narrowing towards its distal end, so that when extended in the safety position, a fmger cannot be accidentally inserted into the sheath opening.

Accordingly, it will be appreciated by persons skilled in the art that numerous variations and/or modifications may be made to the invention without departing from the scope of the invention as broadly described. The present embodiments are, therefore, to be considered in all respects as illustrative and not restrictive.

## Claims

1. A multi-compartment syringe (210) adapted for storage and subsequent ejection of a mixed substance (225) from an exit portion (216) thereof, the multi-compartment syringe (210) comprising:
a first compartment (211) adapted for sealed storage of a first substance (212);
a second compartment (213) adapted for sealed storage of a second substance (214);
communication means having a sealed state and a communicating substance state, the communication means comprising a piercing element (221) arranged for piercing a slidable sealing element (218);
communication actuation means (223) adapted to move the communication means from the sealed state to the communicating state;
first urging means (223) by which the second substance (214) in at least the second compartment (213), is urged from the second compartment (213) into the first compartment (211) so as to mix with the first substance (212) thereby to form the mixed substance (225); and
second urging means (223) by which the mixed substance (225) in the first compartment (211) is urged through the exit portion (216),
**characterised in that** the piercing element (221) has a single tip arranged for piercing a single slidable sealing element (218) and also being **characterised in that** the communication means further includes a breakable seal (227) for sealing substance flow through the piercing means (221), whereby, when in the communicating substance state, the slidable seal (218) is pierced by the piercing element (221) and the breakable seal (227) is broken so as to permit the second compartment (213) to be placed in substance communication with the first compartment (211).

2. A syringe according to claim 1 wherein the first and second urging means comprise a single plunger (223) aligned with a longitudinal axis of the syringe (210), the plunger (223) in a first range of movement acting as the first urging means, and the plunger (223) in a second range of movement acting as the second urging means.

3. A syringe according to claim 1 or 2 wherein the communication means comprises a hollow needle (221) adapted for piercing interconnection of the first compartment (211) with the second compartment (213).

4. A syringe according to any one of the preceding claims wherein the first compartment (211), the second compartment (213), the communication means, the first urging means (223) and the second urging means (223) are all aligned along the longitudinal axis of the syringe (210).

5. A syringe according to any one of the preceding claims wherein the first compartment (211) is defined within a syringe housing of the syringe (210).

6. A syringe according to claim 5 wherein the second compartment (213) is slidably received by the syringe housing.

7. A syringe according to any one of the preceding claims wherein the communication means further comprises an intermediate volume (224) that reduces to substantially zero as the communication means moves from the sealed state to the communicating substance state.

8. A method for operating a multi-compartment syringe (210), comprising:
providing a first cylindrical storage means (211) and a second cylindrical storage means (213), the second storage means (213) being received by the first storage means (211) and having a single tipped piercing element (221) at one end region thereof and a slidable sealing element (222) at an opposite second end region thereof, and a breakable seal (227) sealing the piercing element (221) from an internal storage volume of the second storage means (213);
driving the second storage means (213) axially such that the single tipped piercing element (221) pierces a single sealing element (218) of the first cylindrical storage means;
driving the sealing element (222) of the second cylindrical storage means (213) axially to breach the breakable seal (227) of the second storage means (213) thereby placing the first and second storage means (211 and 213) in fluid communication for discharge of contents of the second storage means (213) into the first storage means (211); and
discharging the contents (214) of the second storage means (213) into the first storage means (211) to provide a mixed substance (225).

9. A method according to claim 8 wherein the syringe (210) comprises an actuator (223) for driving the sealing element (222) of the second cylindrical storage means (213) axially to breach the breakable seal (227) of the second storage means (213), and wherein the contents (214) of the second storage means (213) are discharged into the first storage means (211) by driving the sealing element (222) of the second storage means (213) axially using the actuator (223).

10. A method according to claim 9 wherein the actuator comprises a plunger (223) adapted for releasable engagement with the sealing element (222) of the second storage means (213).

11. A method according to claim 10 wherein the plunger (223) is detachable from the sealing element (222) of the second storage means (213) such that the plunger (223) is substantially prevented from withdrawing the sealing element (222) from the second storage means (213).

12. A method according to claim 10 or 11 wherein the plunger (223) includes a movable barrier (52) which acts to limit actuation of the sealing element (222) of the second storage means (213) by the plunger (223) to a preload movement, whereby movement of the plunger (223) relative to the first storage means (211) is stopped at a preload position.

13. A method according to claim 12 wherein the plunger (223) is dimensioned to drive the sealing element (222) of the second storage means (213) to the second end region thereof for discharge of the contents (214) of the second storage means (213) into the first storage means (211) when the movable barrier (52) is set to a non-limiting position.

14. A method according to any one of claims 8 to 13 wherein the syringe (210) comprises a needle assembly (225) for delivery of the mixed substance from the first storage means (211) and a safety sheath (314) operable between a first locked extended position so as to shroud a needle tip (319) of the needle (318) and a second locked retracted position so as to expose the needle tip (319) for use, the sheath (314) being prevented from axial movement when in the locked extended position.

15. A method according to any one of claims 8 to 14 wherein the piercing element (221) is a hollow needle through which the contents (214) of the second storage means (213) pass into the first storage means (211).

## Patentansprüche

1. Die Erfindung betrifft eine Mehrkammerspritze (210) zur Speicherung und anschließenden Ejektion eines Substanzgemisches (225) aus einem Abgangsbereich (216) derselben, wobei die Mehrkammerspritze (210) folgende Bestandteile umfasst:
eine erste Kammer (211) für das dichte Aufbewahren einer ersten Substanz (212);
eine zweite Kammer (213) für das dichte Aufbewahren einer zweiten Substanz (214);
Verbindungselement in einem dichten Zustand sowie einem Verbindungszustand zwischen Substanzen; das Verbindungselement umfasst ein Einstichelement (221) für den Einstich in ein verschiebbares Dichtungselement (218);
eine Betätigungseinrichtung für die Verbindung (223), anhand der das Verbindungselement vom dichten Zustand in den Verbindungszustand gebracht werden kann;
eine erste Treibeinrichtung (223), durch die die zweite Substanz (214) zumindest in der zweiten Kammer (213) von der zweiten Kammer (213) in die erste Kammer (211) getrieben werden kann, um sie mit der ersten Substanz (212) zu vermischen und so das Substanzgemisch zu erhalten (225); und
eine zweite Treibeinrichtung (223), durch die das Substanzgemisch (225) in der ersten Kammer (211) durch den Ausgangsabschnitt (216) getrieben wird,
**dadurch gekennzeichnet, dass** das Einstichelement (221) über eine einzige Spitze verfügt, mit der ein einziges verschiebbares Dichtungselement (218) durchstochen wird, und ferner **dadurch gekennzeichnet, dass** das Verbindungselement außerdem einen zerbrechlichen Verschluss (227) zum Fluss der Substanz durch das Einstichelement (221) umfasst, wobei der verschiebbare Verschluss (218) vom Einstichelement (221) durchstoßen und der zerbrechliche Verschluss (227) durchbrochen wird, um die Verbindung der Substanzen der zweiten Kammer (213) und der ersten Kammer (211) zu erzielen.

2. Eine Spritze gemäß Anspruch 1, wobei die erste und die zweite Treibeinrichtung einen einzigen Kolben (223) entlang der Längsachse der Spritze (210) umfassen und der Kolben (223) in einem ersten Bewegungsablauf als die erste Treibeinrichtung und in einem zweiten Bewegungsablauf als die zweite Treibeinrichtung fungiert.

3. Eine Spritze gemäß Anspruch 1 oder 2, wobei das Verbindungselement eine Hohlnadel (221) enthält, deren Funktion es ist, eine Einstichverbindung der ersten Kammer (211) und der zweiten Kammer (213) herzustellen.

4. Eine Spritze gemäß eines der vorangehenden Ansprüche, wobei die erste Kammer (211), die zweite Kammer (213), das Verbindungselement, das erste Treibelement (223) und das zweite Treibelement (223) entlang der Längsachse der Spritze (210) angeordnet sind.

5. Eine Spritze gemäß einem der vorangehenden 5 Ansprüche, wobei die erste Kammer (211) innerhalb eines Kanülengehäuses der Spritze (210) angeordnet ist.

6. Eine Spritze gemäß Anspruch 5, wobei die zweite Kammer (213) verschieblich vom Kanülengehäuse aufgenommen ist.

7. Eine Spritze gemäß eines der vorangehenden Ansprüche, wobei das Verbindungselement weiterhin einen Zwischenraum (224) beinhaltet, der durch Bewegen des Verbindungselements vom dichten in den Verbindungszustand der Substanz auf beinahe Null gebracht wird.

8. Ein Bedienungsverfahren einer Mehrkammerspritze (210) mit:
einer ersten zylinderförmigen Speicherkammer (211) und einer zweiten zylinderförmigen Speicherkammer (213), wobei die erste Speicherkammer (211) die zweite Speicherkammer (213) aufnimmt und erstere ein einziges geneigtes Einstichelement (221) an einem Endbereich und ein verschiebbares Dichtungselement (222) auf dem gegenüberliegenden, zweiten Endbereich sowie einen zerbrechlichen Verschluss (227) zu einem inneren Speichervolumen der zweiten Speicherkammer (213) aufweist;
axialer Antrieb des zweiten Speichervolumens (213), so dass das geneigte Einstichelement (221) ein einzelnes Dichtungselement (218) der ersten zylinderförmigen Speicherkammer durchsticht;
axialer Antrieb des Dichtungselements (222) der zweiten zylinderförmigen Speicherkammer (213), um den zerbrechlichen Verschluss (227) der zweiten Speicherkammer (213) zu durchbrechen, wodurch zwischen der ersten und der zweiten Speicherkammer (211 und 213) eine Verbindung der flüssigen Substanzen zur anschließenden Abgabe des Inhalts der zweiten Speicherkammer (213) in die erste Speicherkammer (211) hergestellt wird; und
Abgabe des Inhalts (214) der zweiten Speicherkammer (213) in die erste Speicherkammer (211), um ein Substanzgemisch (225) zu erhalten.

9. Ein Verfahren gemäß Anspruch 8, wobei die Spritze (210) einen Auslöser (223) zur axialen Bewegung des Dichtungselements (222) der zweiten zylinderförmigen Speicherkammer (213) umfasst, um den zerbrechlichen Verschluss (227) der zweiten Speicherkammer (213) zu durchbrechen, und wobei der Inhalt (214) der zweiten Speicherkammer (213) durch Bewegen des Dichtungselements (222) derselben unter axialer Betätigung des Auslösers (223) in die erste Speicherkammer (211) abgegeben wird.

10. Ein Verfahren gemäß Anspruch 9, wobei der Auslöser einen Kolben (223) für einen lösbaren Eingriff mit dem Dichtungselement 5 (222) der zweiten Speicherkammer (213) enthält.

11. Ein Verfahren gemäß Anspruch 10, wobei der Kolben (223) vom Verbindungselement (222) der zweiten Speicherkammer (213) gelöst werden kann, so dass ein Herausziehen des Verbindungselements (222) aus der zweiten Speicherkammer (213) durch den Kolben (223) weitgehend verhindert wird.

12. Ein Verfahren gemäß Anspruch 10 oder 11, wobei der Kolben (223) eine bewegliche Schranke (52) beinhaltet, die die Betätigung des Dichtungselements (222) der zweiten Speicherkammer (213) durch den Kolben (223) auf eine vorladende Bewegung eingrenzt und durch die die Bewegung des Kolbens (223) zur ersten Speicherkammer (211) bei einer vorladenden Bewegung beendet wird.

13. Ein Verfahren gemäß Anspruch 12, wobei der Kolben (223) so dimensioniert ist, dass das Dichtungselement (222) der zweiten Speicherkammer (213) zu dessen zweiten Endbereich bewegt wird, um deren Inhalt (214) in die erste Speicherkammer (211) abzugeben, wenn die bewegliche Schranke (52) in eine nicht-begrenzende Position gebracht wird.

14. Eine Methode gemäß einem der Ansprüche 8 bis 13, wobei die Spritze (210) eine Nadelanordnung (225) zur Abgabe des Substanzgemisches aus der ersten Speicherkammer (211) und eine Schutzhülle (314) umfasst, die zwischen einer ersten verriegelten, ausgefahrenen Stellung zum Bedecken einer Nadelspitze (319) der Nadel (318) und einer zweiten verriegelten, eingefahrenen Stellung zur Freilegung der Nadelspitze (319) zu deren Verwendung eingesetzt werden kann, wobei die Schutzhülle (314) in der verriegelten, ausgefahrenen Stellung nicht axial bewegt werden kann.

15. Ein Verfahren gemäß einem der Ansprüche 8 bis 14, wobei das Einstichelement (221) in einer Hohlnadel besteht, durch die der Inhalt (214) der zweiten Speicherkammer (213) in die erste Speicherkammer (211) abgegeben wird.

## Revendications

1. Seringue multi-compartiments (210) adaptée au stockage et à l'éjection ultérieure d'une substance mélangée (225) depuis une section de sortie (216) de celle-ci, la seringue multi-compartiments (210) comprenant :
un premier compartiment (211) adapté au stockage hermétique d'une première substance (212) ;
un deuxième compartiment (213) adapté au stockage hermétique d'une deuxième substance (214) ;
un moyen de communication ayant un état scellé et un état de communication de substances, le moyen de communication comprenant un élément de perçage (221) disposé de façon à percer un élément de scellement coulissant (218) ;
un moyen d'activation de communication (223) adapté pour faire passer le moyen de communication de l'état scellé à l'état de communication ;
un premier moyen d'impulsion (223) permettant à la deuxième substance (214) dans au moins dans le deuxième compartiment (213) d'être expulsée du deuxième compartiment (213) vers le premier compartiment (211) afin de se mélanger à la première substance (212) pour former ainsi la substance mélangée (225) ; et
un deuxième moyen d'impulsion (223) permettant à la substance mélangée (225) dans le premier compartiment (211) d'être expulsée via la section de sortie (216),
**se caractérisant en ce que** l'élément de perçage (221) possède un embout unique disposé de façon à percer un élément de scellement coulissant unique (218) et se caractérisant également **en ce que** le moyen de communication comprend en outre un joint cassable (227) destiné à sceller l'écoulement de substance à travers le moyen de perçage (221), moyennant quoi, lorsqu'il se trouve dans l'état de communication de substances, le joint coulissant (218) est percé par l'élément de perçage (221) et le joint cassable (227) est rompu pour permettre au deuxième compartiment (213) d'être placé en communication de substances avec le premier compartiment (211).

2. Seringue selon la revendication 1, dans laquelle les premier et deuxième moyens d'impulsion comprennent un piston unique (223) aligné sur un axe longitudinal de la seringue (210), le piston (223) dans une première plage de mouvement agissant en tant que premier moyen d'impulsion, et le piston (223) dans une deuxième plage de mouvement agissant en tant que deuxième moyen d'impulsion.

3. Seringue selon la revendication 1 ou 2, dans laquelle le moyen de communication se compose d'une aiguille creuse (221) adaptée au perçage de l'interconnexion entre le premier compartiment (211) et le deuxième compartiment (213).

4. Seringue selon l'une quelconque des revendications précédentes, dans laquelle le premier compartiment (211), le deuxième compartiment (213), le moyen de communication, le premier moyen d'impulsion (223) et le deuxième moyen d'impulsion (223) sont tous alignés le long de l'axe longitudinal de la seringue (210).

5. Seringue selon l'une quelconque des revendications précédentes, dans laquelle le premier compartiment (211) est défini dans un logement de seringue de la seringue (210).

6. Seringue selon la revendication 5, dans laquelle le deuxième compartiment (213) est reçu de manière coulissante par le logement de seringue.

7. Seringue selon l'une quelconque des revendications précédentes, dans laquelle le moyen de communication comprend en outre un volume intermédiaire (224) qui s'abaisse pratiquement jusqu'à zéro lorsque le moyen de communication passe de l'état scellé à l'état de communication de substances.

8. Procédé d'utilisation d'une seringue multi-compartiments (210), comprenant :
la mise à disposition d'un premier moyen de stockage cylindrique (211) et d'un deuxième moyen de stockage cylindrique (213), le deuxième moyen de stockage (213) étant reçu par le premier moyen de stockage (211) et possédant un élément de perçage à embout unique (221) à une extrémité de celui-ci et un élément de scellement coulissant (222) à une deuxième extrémité opposée de celui-ci, et un joint cassable (227) scellant l'élément de perçage (221) à partir d'un volume de stockage interne du deuxième moyen de stockage (213) ;
l'entraînement du deuxième moyen de stockage (213) axialement de telle sorte que l'élément de perçage à embout unique (221) perce un élément de scellement unique (218) du premier moyen de stockage cylindrique ;
l'entraînement de l'élément de scellement (222) du deuxième moyen de stockage cylindrique (213) axialement pour rompre le joint cassable (227) du deuxième moyen de stockage (213), plaçant ainsi les premier et deuxième moyens de stockage (211 et 213) en communication fluidique pour décharger le contenu du deuxième moyen de stockage (213) dans le premier moyen de stockage (211) ; et
le déchargement du contenu (214) du deuxième moyen de stockage (213) dans le premier moyen de stockage (211) pour fournir une substance mélangée (225).

9. Procédé selon la revendication 8, dans lequel la seringue (210) comprend un actionneur (223) pour l'entraînement de l'élément de scellement (222) du deuxième moyen de stockage cylindrique (213) axialement pour rompre le joint cassable (227) du deuxième moyen de stockage (213), et dans lequel le contenu (214) du deuxième moyen de stockage (213) est déchargé dans le premier moyen de stockage (211) en entraînant l'élément de scellement (222) du deuxième moyen de stockage (213) axialement à l'aide de l'actionneur (223).

10. Procédé selon la revendication 9, dans lequel l'actionneur comprend un piston (223) adapté à l'engagement libérable avec l'élément de scellement (222) du deuxième moyen de stockage (213).

11. Procédé selon la revendication 10, dans lequel le piston (223) peut être détaché de l'élément de scellement (222) du deuxième moyen de stockage (213) de façon à empêcher sensiblement que le piston (223) retire l'élément de scellement (222) du deuxième moyen de stockage (213).

12. Procédé selon la revendication 10 ou 11, dans lequel le piston (223) comprend une barrière mobile (52) servant à limiter l'actionnement de l'élément de scellement (222) du deuxième moyen de stockage (213) par le piston (223) à un mouvement de préchargement, moyennant quoi le mouvement du piston (223) par rapport au premier moyen de stockage (211) est arrêté en position de préchargement.

13. Procédé selon la revendication 12, dans lequel le piston (223) est dimensionné de façon à entraîner l'élément de scellement (222) du deuxième moyen de stockage (213) vers la deuxième extrémité de celui-ci pour le déchargement du contenu (214) du deuxième moyen de stockage (213) dans le premier moyen de stockage (211) lorsque la barrière mobile (52) est réglée sur une position non limitative.

14. Procédé selon l'une quelconque des revendications 8 à 13, dans lequel la seringue (210) comprend un ensemble aiguille (225) pour l'administration de la substance mélangée dans le premier moyen de stockage (211) et une gaine de sécurité (314) utilisable entre une première position déployée verrouillée afin d'envelopper un embout d'aiguille (319) de l'aiguille (318) et une deuxième position rétractée verrouillée afin d'exposer l'embout de l'aiguille (319) pour l'utilisation, la gaine (314) étant empêchée d'effectuer un mouvement axial lorsqu'elle est en position déployée verrouillée.

15. Procédé selon l'une quelconque des revendications 8 à 14, dans lequel l'élément de perçage (221) est une aiguille creuse à travers laquelle le contenu (214) du deuxième moyen de stockage (213) passe dans le premier moyen de stockage (211).
